# EUROPEAN PATENT SPECIFICATION

(11) **EP 1 210 953 B1**
(45) Date of publication and mention of the grant of the patent: **30.06.2004**
(21) Application number: 01128358.7
(22) Date of filing: 30.11.2001
(51) Int. Cl.: A61L 31/04

(54) **Endotracheal tube**
Endotrachealtubus
Tube endotrachéal

(30) Priority: 30.11.2000 JP 2000364424
(43) Date of publication of application: 05.06.2002
(60) Divisional application: 03011119.9
(73) Proprietor: KURARAY CO., LTD., Kurashiki-shi, Okayama-ken 710 (JP)
(72) Inventor: Ogushi, Masayasu, Tsukuba-shi, Ibaraki-ken (JP); Fukuda, Motohiro, Tsukuba-shi, Ibaraki-ken (JP); Zento, Toshiyuki, Tsukuba-shi, Ibaraki-ken (JP); Kirita, Yasuzo, Osaka-shi, Osaka-fu (JP); Nakashima, Toshihide, Kurashiki-shi, Okayama-ken (JP); Fujieda, Yukihiro, Kurashiki-shi, Okayama-ken (JP)
(74) Representative: HOFFMANN - EITLE

(56) References cited:
- WO-A-01/94466
- WO-A-99/37338
- GB-A- 2 108 971
- US-A- 4 616 064
- DATABASE WPI Section Ch, Week 199820 Derwent Publications Ltd., London, GB; Class A18, AN 1998-225233 XP002194181 & JP 10 067894 A (KURARAY CO LTD), 10 March 1998 (1998-03-10)

## Description

### Field of the Invention

The present invention relates to an endotracheal tube. More specifically, the present invention relates to an endotracheal tube which can be suitably used for an orally inserted endotracheal tube, a nasally inserted endotracheal tube, and a tube for tracheostomy to be inserted into the trachea from a tracheostoma.

The endotracheal tube of the present invention is not composed of a plasticized-polyvinyl chloride, and is excellent in kink resistance, slidability and prevention of sticking.

### Discussion of the Related Art

An endotracheal tube is a medical tool used for an anesthetic treatment during surgery. A plasticized-polyvinyl chloride has been used in many of endotracheal tubes in consideration of mechanical strength, transparency and costs, as well as giving appropriate flexibility. However, the plasticized-polyvinyl chloride may generate a harmful substance such as dioxin when the plasticized-polyvinyl chloride is burned. Also, since a plasticizer such as dioctyl phthalate incorporated into the plasticized polyvinyl chloride is regarded as a harmful substance in the environment, the plasticized-polyvinyl chloride is not preferable for medical tools.

As an endotracheal tube which is not composed of a plasticized-polyvinyl chloride, an endotracheal tube composed of a silicone resin has been proposed. However, the silicone resin is expensive, and a crosslinking process is required for the production of the silicone resin. Therefore, the endotracheal tube would become expensive.

In view of these defects, Japanese Patent Laid-Open No. Hei 10-67894 discloses a resin composition comprising a styrenic elastomer and a polypropylene as a material for a medical tool. This resin composition is excellent in flexibility and transparency, and has heat resistance durable for autoclaving and biocompatibility.

However, there are some defects in the resin composition to be improved for its use as an endotracheal tube.

For instance, there have been required for the tube to have kink resistance for preventing the tube from being collapsed even when the tube is inserted into the trachea and allowed to curve, and slidability in order that a suction catheter for removing excretion accumulated in the internal part of the trachea can be smoothly inserted. There has been proposed to wind a helical steel wire onto the endotracheal tube made of this resin composition. However, there are some defects in this tube such that its production steps are complicated, and that the endotracheal tube becomes expensive. Moreover, noncombustible substances such as a metal would be disposed as wastes into an incinerator.

Also, there are some defects in the resin composition such that it would be difficult to produce a cuff by blow molding of the resin composition, so that when the cuff is provided in the endotracheal tube, the prevention of sticking required for uniformly expanding a cuff cannot be sufficiently exhibited.

As a tube being not made of a polyvinyl chloride onto which helical steel wire is not wound, Japanese Patent Laid-Open Nos. Hei 9-75443 and Hei 11-151293 disclose a multi-layer tube. However, there are some defects in the multi-layer tube such that the tube is produced by coextrusion to have multiple layers, so that its production steps are complicated.

An object of the present invention is to provide an endotracheal tube excellent in kink resistance, slidability and prevention of sticking.

These and other objects of the present invention will be apparent from the following description.

### SUMMARY OF THE INVENTION

According to the present invention, there is provided an endotracheal tube comprising a tube obtainable by subjecting a resin composition comprising a styrenic elastomer and a polyolefin to extrusion-molding, wherein the tube has a storage modulus (MD) of 5.0 x 10⁶ to 8.0 x 10⁷ N/m² (5.0 x 10⁷ to 8.0 x 10⁸ dyne/cm²) in the extrusion direction at 25°C, and has a ratio of the storage modulus (MD) in the extrusion direction to a storage modulus (TD) in the circumferential direction (MD/TD) of not more than 1.3 at 25°C, the weight ratio of the polyolefin to the styrenic elastomer being 20/80 to 40/60.

Preferably, the endotracheal tube is provided with a cuff obtainable by subjecting a resin composition comprising a styrenic elastomer and a polyolefin to blow-molding on an outer peripheral surface, the weight ratio of the styrenic elastomer to the polyolefin being 60/40-80/20, the cuff having a storage modulus of not more than 5.0 x 10⁷ N/m² (5.0 x 10⁸ dyne/cm²) at 25°C, and the resin composition constituting the cuff having a melt tension of not less than 1 g at 230°C.

### BRIEF DESCRIPTION OF THE DRAWINGS

Figure 1 is a schematic explanatory view showing one embodiment of an endotracheal tube having no cuff of the present invention;
Figure 2 is a schematic explanatory view showing one embodiment of an endotracheal tube comprising a cuff of the present invention;
Figure 3 is a schematic explanatory view showing a kink of a tube;
Figure 4 is a schematic explanatory view an extrusion blow molding machine used in each Example and each Comparative Example; and
Figure 5 is a schematic explanatory view of a cuff obtained in each of Examples 8 to 11 and Comparative Examples 5 and 6.

### DETAILED DESCRIPTION OF THE INVENTION

In the endotracheal tube of the present invention, a tube obtained by subjecting a resin composition comprising a styrenic elastomer and a polyolefin to extrusion-molding is used as a main tube.

As the polyolefin, various polyolefins made of olefin monomers can be used. The polyolefin includes, for instance, polyethylenes such as high-density polyethylene, low-density polyethylene, linear low-density polyethylene and high pressure processed ethylene-α-olefin copolymer; polypropylenes such as propylene homopolymer; a random copolymer of ethylene and propylene; a block-type polypropylene comprising ethylene blocks; terpolymers of propylene, ethylene and butene-1; and the like. Each of those polyolefins can be used alone or in admixture of at least two kinds. Among those polyolefins, the polypropylenes are especially preferable.

As to the melt viscosity of the polyolefin, the melt flow rate (MFR) of the polyolefin is within the range of preferably 0.1 to 500, more preferably 2 to 200, as determined by the method in accordance with ASTM D-1238 at 230°C under the load of 2160 g.

It is preferable that the styrenic elastomer is a block copolymer of a styrenic polymer block (A) and a hydrogenated conjugated diene polymer block (B).

The styrenic polymer block (A) is made of a styrenic monomer. Examples of the styrenic monomer are, for instance, styrene, α-methylstyrene, 3-methylstyrene, 4-propylstyrene, 4-cyclohexylstyrene, 4-dodecylstyrene, 2-ethyl-4-benzylstyrene, 4-(phenylbutyl)styrene, and the like. Those monomers can be used alone or in admixture of at least two kinds. Among those styrene monomers, styrene is preferable.

The number-average molecular weight of the styrenic polymer block (A) is not limited to specified ones. It is preferable that the number-average molecular weight is within the range of 2500 to 20000.

The content of the styrenic polymer block (A) in the block copolymer is preferably not less than 10% by weight, more preferably not less than 15% by weight, from the viewpoint of improving the mechanical strength of the block copolymer. Also, the content of the styrenic polymer block (A) in the block copolymer is preferably not more than 40% by weight, more preferably not more than 30% by weight, from the viewpoint of facilitating homogeneous mixing with the polyolefin. Accordingly, from these viewpoints, the content of the styrenic polymer block (A) in the block copolymer is preferably 10 to 40% by weight, more preferably 15 to 30% by weight.

It is preferable that the hydrogenated conjugated diene polymer block (B) has at least one polymer block selected from the group consisting of a hydrogenated polyisoprene block (B1), a hydrogenated isoprene/butadiene copolymer block (B2) and a hydrogenated polybutadiene block (B3), from the viewpoint of the balance of flexibility and economics.

It is preferable that the hydrogenated polyisoprene block (B1) is a hydrogenated polyisoprene block made of a polyisoprene having a 1,2-bond and 3,4-bond content (hereinafter simply referred to as "content of vinyl bonds") of 10 to 75% by mol, in which not less than 70% of carbon-carbon double bonds of the polyisoprene are hydrogenated.

The content of vinyl bonds in the hydrogenated polyisoprene block (B1) is preferably not less than 10% by mol, more preferably not less than 20% by mol, from the viewpoint of increasing the transparency of an endotracheal tube. Also, the content of vinyl bonds is preferably not more than 75% by mol, more preferably not more than 65% by mol, from the viewpoint of not making a glass transition temperature (Tg) of the hydrogenated polyisoprene block (B1) exceedingly high, thereby giving the endotracheal tube appropriate flexibilities. Accordingly, from these viewpoints, the content of vinyl bonds in the hydrogenated polyisoprene block (B1) is preferably 10 to 75% by weight, more preferably 20 to 65% by weight.

In addition, the ratio of hydrogenation of carbon-carbon double bonds of the polyisoprene is preferably not less than 70%, more preferably not less than 80% by mol, from the viewpoints of increasing its compatibility with the polyolefin, thereby giving the endotracheal tube excellent transparency.

The number-average molecular weight of the hydrogenated polyisoprene block (B1) is not limited to specified ones. It is preferable that the number-average molecular weight is within the range of 10000 to 200000.

It is preferable that the hydrogenated isoprene/butadiene copolymer block (B2) is a hydrogenated isoprene/butadiene copolymer block comprising an isoprene/butadiene copolymer having a 1,2-bond and 3,4-bond content, i.e. the content of vinyl bonds, of 20 to 85% by mol, obtained by copolymerizing isoprene and butadiene in a weight ratio of 5/95 to 95/5, wherein not less than 70% of carbon-carbon double bonds are hydrogenated.

The weight ratio of isoprene/butadiene is preferably not more than 95/5, more preferably not more than 80/20, from the viewpoints of not making the glass transition temperature (Tg) exceedingly high when the content of vinyl bonds in the hydrogenated isoprene/butadiene copolymer block (B2) is not less than 75% by mol, and increasing the flexibility of the endotracheal tube. Also, the weight ratio of isoprene/butadiene is preferably not less than 5/95, more preferably not less than 20/80, from the viewpoint of increasing the transparency of the endotracheal tube when the content of vinyl bonds in the hydrogenated isoprene/butadiene copolymer block (B2) is less than 30% by mol. Accordingly, from these viewpoints, the weight ratio of isoprene/butadiene is preferably 5/95 to 95/5, more preferably 20/80 to 80/20.

It is desired that carbon-carbon double bonds of the isoprene/butadiene copolymer are hydrogenated in a ratio of not less than 70%, preferably not less than 80%, from the viewpoints of increasing its compatibility with the polyolefin and improving the transparency of the endotracheal tube.

The content of vinyl bonds in the hydrogenated polyisoprene/butadiene copolymer block is preferably not less than 20% by mol, more preferably not less than 40% by mol, from the viewpoint of increasing the transparency of the endotracheal tube. Also, the content of vinyl bonds is preferably not more than 85% by mol, more preferably not more than 70% by mol, from the viewpoints of not making a glass transition temperature (Tg) of the hydrogenated polyisoprene block (B1) exceedingly high, thereby giving the endotracheal tube appropriate flexibilities. Accordingly, from these viewpoints, the content of vinyl bonds in the hydrogenated polyisoprene/butadiene copolymer block is preferably 20 to 85% by weight, more preferably 40 to 70% by weight.

The polymerization form of isoprene and butadiene in the hydrogenated isoprene/butadiene copolymer block (B2) is not limited to specified ones, and can be any of random, block and tapered forms.

The number-average molecular weight of the hydrogenated isoprene/butadiene copolymer block (B2) is not limited to specified ones. It is preferable that the number-average molecular weight is within the range of 10000 to 200000.

It is preferable that the hydrogenated polybutadiene block (B3) is a hydrogenated polybutadiene block made of a polybutadiene having a 1,2-bond and 3,4-bond content, i.e. the content of vinyl bonds, of not less than 45% by mol, wherein not less than 70% of carbon-carbon double bonds of the polybutadiene are hydrogenated.

The content of vinyl bonds in the polybutadiene is preferably not less than 45% by mol, more preferably 60 to 80% by mol, from the viewpoint of increasing the transparency of the endotracheal tube.

The ratio of hydrogenation of carbon-carbon double bonds of the polybutadiene is preferably not less than 70%, more preferably not less than 80% by mol, from the viewpoints of increasing its compatibility with the polyolefin, thereby increasing transparency of the endotracheal tube.

The number-average molecular weight of the hydrogenated polybutadiene block (B3) is not limited to specified ones. The number-average molecular weight is preferably within the range of 10000 to 200000.

The content of the hydrogenated conjugated polymer block (B) in the block copolymer is preferably not more than 90% by weight, more preferably not more than 85% by weight, from the viewpoint of improving mechanical strength of the block copolymer, and the content is preferably not less than 60% by weight, more preferably not less than 70% by weight, from the viewpoint of facilitating homogeneous mixing of the block copolymer with the polyolefin. Accordingly, from these viewpoints, the content of the hydrogenated conjugated diene polymer block (B) in the block copolymer is preferably 60 to 90% by weight, more preferably 70 to 85% by weight.

In the block copolymer of the styrenic polymer block (A) and the hydrogenated conjugated diene polymer (B), the bonding forms of the styrenic polymer block (A) and the hydrogenated conjugated diene polymer block (B) are not limited to specified ones, and can be any of linear form, branched form and a given combination of these.

When the styrenic polymer block (A) is represented by "A", and the hydrogenated conjugated diene polymer block (B) is represented by "B", the molecular structure of the block copolymer includes the forms such as A-(B-A)ₙ and (A-B)ₙ, wherein n is an integer of not less than 1. The molecular structure of the block copolymer before hydrogenation may have a star-like form together with a coupling agent such as divinylbenzene, a tin compound or a silane compound, such as (A-B)ₘX, wherein m is an integer of not less than 2, and X is a residue of a coupling agent.

As the block copolymer, those having the above-mentioned molecular structures can be used alone, or in admixture of at least two different molecular structures, such as a mixture of triblock-type and diblock-type.

The number-average molecular weight of the block copolymer is not limited to specified ones. The number-average molecular weight is preferably within the range of 30000 to 300000.

The endotracheal tube of the present invention comprises a tube obtained by subjecting the resin composition comprising a styrenic elastomer and a polyolefin to extrusion-molding.

The tube has a storage modulus of 5.0 × 10⁶ - 8.0 × 10⁷ N/m² (5.0 × 10⁷ to 8.0 × 10⁸ dyne/cm²) in the extrusion direction (MD) at 25°C, and a ratio of the storage modulus in the extrusion direction (MD) to a storage modulus in the circumferential direction (TD), i.e. MD/TD, of not more than 1.3 at 25°C.

The storage modulus can be determined by using a general dynamic viscoelasticity analyzer, for instance, Rheospectra commercially available from Rheology under the trade mane of DVE-V4 FT Rheospectra and the like.

The tube has a storage modulus in the extrusion direction (MD) of not less than 5.0 × 10⁶ N/m² (5.0 × 10⁷ dyne/cm²), preferably not less than 7.0 × 10⁶ N/m² (7.0 × 10⁷ dyne/cm²), more preferably not less than 8.0 × 10⁶ N/m² (8.0 × 10⁷ dyne/cm²) at 25°C, from the viewpoint of preventing the tube from being too flexible, thereby facilitating insertion of the tube into the trachea. Also, the tube has a storage modulus in the extrusion direction (MD) of not more than 8.0 × 10⁷ N/m² (8.0 × 10⁸ dyne/cm²), preferably not more than 4.0 × 10⁷ N/m² (4.0 × 10⁸ dyne/cm²), more preferably not more than 2.0 × 10⁷ N/m² (2.0 × 10⁸ dyne/cm²) at 25°C, from the viewpoint of preventing the tube from becoming too rigid, thereby avoiding the generation of damages in the trachea. Accordingly, from the viewpoints, the tube has a storage modulus in the extrusion direction (MD) of 5.0 × 10⁶ - 8.0 × 10⁷ N/m² (5.0 × 10⁷ to 8.0 × 10⁸ dyne/cm²), preferably 7.0 × 10⁶ - 4.0 × 10⁷ N/m² (7.0 × 10⁷ to 4.0 × 10⁸ dyne/cm²), more preferably 8.0 × 10⁶ - 2.0 × 10⁷ N/m² (8.0 × 10⁷ to 2.0 × 10⁸ dyne/cm²).

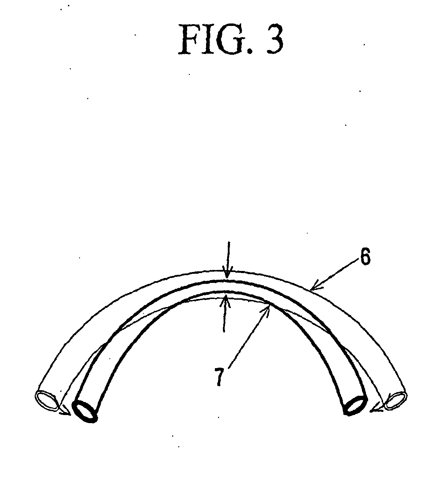

Figure 3 is a schematic explanatory view of a kink of a tube. When the tube is curved, the tensile stress is generated on the long diameter side 6 of the curved tube and the compressive stress is generated on the short diameter side 7 of the tube. These stresses would collapse the tube from a circular form to an oval form. As shown by the solid bold line in Figure 3, when the curvature of the bend of the tube is smaller, the force for collapsing the shape of the cross section of the tube increases, so that the internal space of the tube is finally completely collapsed at the curved portion, to form a kink.

The larger the stresses generated on the long diameter side 6 and the short diameter side 7 of the tube are, the higher the modulus in the extrusion direction of the tube, i.e. the storage modulus (MD), becomes. In order to withstand the force of collapsing the cross section of the tube, it is advantageous that the higher the modulus in the circumferential direction of the tube, i.e. storage modulus (TD), is.

Therefore, idealistically, it is thought that the larger the storage modulus in the circumferential direction of the tube (TD) is, as compared to the storage modulus in the extrusion direction of the tube (MD), the greater the kink resistance becomes.

However, in a case of a tube obtained by subjecting the resin composition comprising a styrenic elastomer and a polyolefin to extrusion-molding, the storage modulus in the extrusion direction (MD) is generally larger than the storage modulus in the circumferential direction (TD) due to the molecular orientation caused by extrusion-molding.

In the present invention, since the ratio of the storage modulus in the extrusion direction of the tube (MD) to the storage modulus in the circumferential direction of the tube (TD), i.e. MD/TD is adjusted to not more than 1.3 at 25°C, excellent kink resistance is imparted to the tube. The ratio of the storage modulus in the extrusion direction of the tube (MD) to the storage modulus in the circumferential direction of the tube (TD), i.e. MD/TD, is preferably not more than 1.2, more preferably not more than 1.1, from the viewpoint of improving the kink resistance of the tube.

The ratio of the storage modulus in the extrusion direction of the tube (MD) to the storage modulus in the circumferential direction (TD), i.e. MD/TD, can be controlled by adjusting the ratio of the polyolefin to the styrenic elastomer.

In order to obtain a tube having excellent kink resistance by imparting the above-mentioned ratio of the storage modulus in the extrusion direction (MD) of the tube to a storage modulus in the circumferential direction of the tube (TD), i.e. MD/TD, to the tube, the weight ratio of the polyolefin to the styrenic elastomer (polyolefin/styrenic elastomer) is adjusted to the range of 20/80 to 40/60, preferably 25/75 to 35/65.

It is preferable that the resin composition constituting the tube contains at least one lubricant selected from the group consisting of fatty acid amide lubricants and fatty acid monoglyceride lubricants in order to improve slidability (operability) when a suction catheter or the like is inserted. The fatty acid amide lubricants and the fatty acid monoglyceride lubricants can be used alone or in admixture of at least two kinds.

The fatty acid amide lubricant includes, for instance, erucic amide, behenic acid amide, oleic amide, stearic acid amide, N-stearyllauric acid amide, N-stearylstearic acid amide, N-stearylbehenic acid amide, N-stearylerucic amide, N-oleyloleic amide, N-oleylbehenic acid amide, N-laurylerucic amide, ethylenebisoleic amide, ethylenebisstearic acid amide, hexamethylenebisoleic amide, hexamethylenebiserucic amide, and the like. Among them, erucic amide, behenic acid amide, oleic amide, stearic acid amide and ethylenebis stearic amide are preferable, and oleic amide is more preferable.

The fatty acid monoglyceride lubricant includes, for instance, lauric acid monoglyceride, myristic acid monoglyceride, palmitic acid monoglyceride, stearic acid monoglyceride, oleic acid monoglyceride, behenic acid monoglyceride, and the like. Among them, stearic monoglyceride is preferable.

The content of the lubricant in the resin composition is preferably not less than 0.05% by weight, from the viewpoint of improving the slidability during insertion of the suction catheter. Also, the content of the lubricant is preferably not more than 0.5% by weight, more preferably not more than 0.2% by weight, from the viewpoint of improvement of printability on the tube surface by avoiding the bleed-out of the lubricant from the tube. Accordingly, from these viewpoints, the content of the lubricant in the resin composition is within the range of preferably 0.05 to 0.5% by weight, more preferably 0.05 to 0.2% by weight.

In the present invention, the endotracheal tube may have a cuff on the outer peripheral surface of the tube.

The cuff can be made of a resin composition comprising a styrenic elastomer and a polyolefin.

As the polyolefin, various polyolefins made of olefinic monomers can be used. The polyolefin includes, for instance, polyethylenes such as high-density polyethylene, low-density polyethylene, linear low-density polyethylene and high pressure processed ethylene-α-olefin copolymer; polypropylenes such as propylene homopolymer; a random copolymer of ethylene and propylene; a block-type polypropylene containing ethylene blocks; terpolymers of propylene, ethylene and butene-1; and the like. These polyolefins can be used alone or in admixture of at least two kinds. Among those polyolefins, a polyolefin in which a cross-linking structure is introduced by applying electron beam irradiation is preferable in order to increase the melt tension during blow-molding,.

As the styrenic elastomer, the same ones as those styrenic elastomers used in the above-mentioned main tube can be exemplified. The styrenic elastomer is preferably a block copolymer suitably used for the above-mentioned tube. In addition, there can be used cross-linked block copolymers prepared by applying electron beam irradiation to the block copolymer, or a cross-linking the block copolymer with a radical such as a peroxide, in order to increase the melt tension during blow-molding.

The resin composition constituting the cuff has a melt tension of not less than 1 g, preferably not less than 1.5 g at 230°C, from the viewpoints of avoidance of the generation of the draw-down of the parison and breakage of the cuff at the draw-up during blow-molding. The melt tension of the resin composition at 230°C is determined by the method described below.

The cuff can be produced by blow-molding the above-mentioned resin composition. When the storage modulus of the cuff at 25°C is too high, the cuff becomes too rigid, so that the cuff which has been shrunk under a pressure of 25 cm H₂O for expanding in a usual trachea would not be sufficiently expanded, and thereby the sealability of the endotracheal tube would be lowered. When the cuff is expanded until the sealing becomes sufficient, the blood capillary in the trachea is pressurized by the internal pressure of the cuff, so that the necrosis of the tissue tends to occur. Therefore, in the present invention, in consideration of these matters, the storage modulus of the cuff is controlled to not more than 5.0 × 10⁷ N/m² (5.0 × 10⁸ dyne/cm²) at 25°C.

The storage modulus can be determined by using the above-mentioned dynamic viscoelasticity analyzer.

The melt tension of the above-mentioned resin composition at 230°C and the storage modulus of the cuff molded therefrom at 25°C can be controlled by adjusting the ratio of the styrenic elastomer to the polyolefin.

In order to satisfy the above-mentioned melt tension of the resin composition at 230°C and the above-mentioned storage modulus of the cuff molded therefrom at 25°C, it is desired that the weight ratio of the styrenic elastomer to the polyolefin (styrenic elastomer/polyolefin) is within the range of 60/40 to 80/20, preferably 70/30 to 80/20.

It is preferable that the resin composition constituting the cuff contains at least one member selected from inorganic fillers and organic cross-linked particles, in order to prevent the cuff from uneven expansion due to sticking during the expansion of the cuff.

The inorganic filler includes, for instance, talc, calcium carbonate, mica, and the like. The organic cross-linked particles include, for instance, cross-linked acrylic resin beads, cross-linked polyurethane beads, cross-linked polystyrene beads and the like. Those inorganic fillers and organic cross-linked particles can be used alone or in admixture of at least two kinds.

The content of at least one member selected from inorganic fillers and organic cross-linked particles in the resin composition constituting the cuff is preferably not less than 5% by weight, from the viewpoint of sufficiently exhibiting the prevention of sticking. Also, the content is preferably not more than 20% by weight, more preferably not more than 10% by weight, from the viewpoint of improving the surface property of the cuff. Accordingly, from these viewpoints, the content is with the range of preferably 5 to 20% by weight, more preferably 5 to 10% by weight.

In the resin composition constituting the above-mentioned tube and cuff, there can be added various additives, including, for instance, an antioxidant, an ultraviolet absorbing agent, a light stabilizer, a colorant, a crystalline nucleus-forming agent, within a range which would not impair the properties of the resin composition. The amount of those additives cannot be absolutely determined because the amount differs depending upon their kinds. It is preferable that the amount of the additive is usually 0.01 to 5 parts by weight based on 100 parts by weight of the resin composition.

To the above-mentioned resin composition, there can be added a softening agent such as a mineral oil, within the range which would not impair the properties of the resin composition. It is preferable that the amount of the softening agent is usually not more than 100 parts by weight based on 100 parts by weight of the resin composition.

Furthermore, there can be added to the above-mentioned resin composition other polymers, including, for instance, hydrogenated polyisoprene, hydrogenated polybutadiene, hydrogenated styrene-butadiene random copolymer, hydrogenated styrene-isoprene random copolymer, ethylene-vinyl acetate copolymer, ethylene-methacrylic acid copolymer, ethylene-acrylic acid copolymer, and their ionomers, ethylene-methyl acrylate copolymer and the like within the range which would not impair the properties of the resin composition.

The endotracheal tube of the present invention can be produced by subjecting the above-mentioned resin composition to extrusion-molding to give a main tube, cutting the main tube to have an appropriate inner diameter satisfying the size prescribed in ISO 5361/1 (Tracheal Tube-Part 1: General Requirement), cutting slantwise one end of the tube, which is to be inserted into a patient at a bevel angle of 38°±10°, rounding off the cut edges by heat treatment, and subjecting the tube to a curving treatment.

When an endotracheal tube having a cuff is produced by extrusion molding, generally, the main tube is provided with a sublumen for passing air through the tube, which is used for expanding a cuff. Thereafter, the cuff is molded into a spindle-like shape or Rugby foot ball-like shape, and bonded to the outer periphery of the main tube at the portion to be inserted to a patient.

It is usually preferable that the main tube is previously provided with a notch communicating with the sublumen for expanding the cuff at the portion to be bonded with the cuff. The endotracheal tube having a cuff is obtained by bonding one end of a tail tube to the sublumen within the size range as defined in ISO 5361/1, and bonding the other end of the tail tube to a pilot balloon, a check valve and the like.

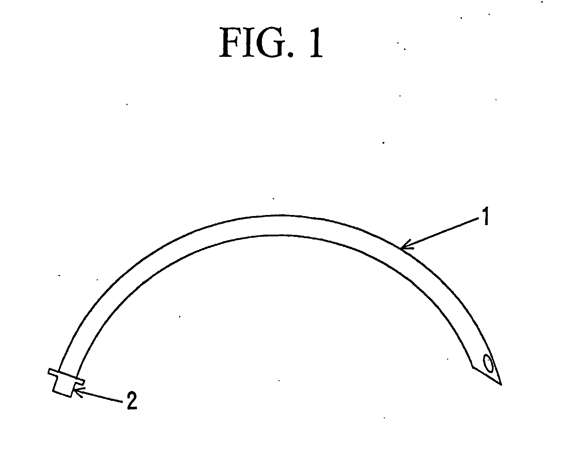

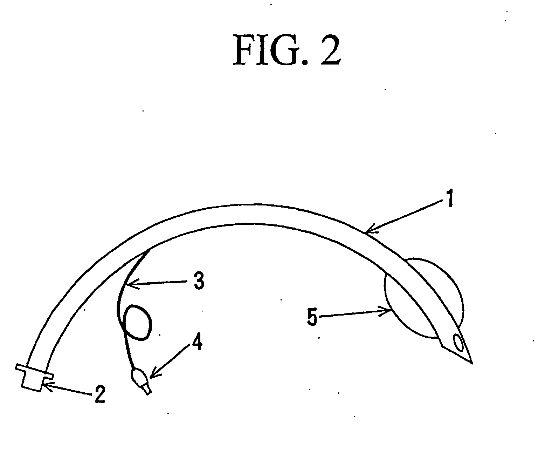

Embodiments of the endotracheal tube produced by the above-mentioned methods are shown in Figure 1 and 2.

Figure 1 is a schematic explanatory view showing one embodiment of an endotracheal tube in which a connector 2 is provided on one end of a main tube 1 not having a cuff.

Figure 2 is a schematic explanatory view showing one embodiment of an endotracheal tube in which a connector 2 is provided on one end of a main tube 1 having a cuff 5. In Figure 2, one end of a tail tube 3 is bonded to a sublumen (not shown) of the main tube 1, and the other end of the tail tube 3 is provided with a pilot balloon 4.

### EXAMPLES

Next, the present invention will be described more specifically on the basis of the examples, without intending to limit the present invention thereto.

The resins, the molding machine, and the determination methods used in Examples and Comparative Examples are as follows.

### [Resin]

- Resin 1:: Polypropylene (random-type, commercially available from Grand Polymer under the trade name of F327)
- Resin 2:: Polypropylene (homo-type, commercially available from Montel under the trade name of SD613)
- Resin 3:: Polyethylene (low-density polyethylene, commercially available from Nippon Polychem K.K. under the trade name of HE30)
- Resin 4:: Styrenic elastomer [hydrogenated SIS (hydrogenated styrene-isoprene-styrene block copolymer), commercially available from Kuraray Co., Ltd. under the trade name ofHYBRA HVS7125, number-average molecular weight 100000, styrene content: 20% by weight, hydrogenation ratio: 90%, content of vinyl bond: 55% by mol]

### [Extruder]

φ40 mm single-screw extruder (commercially available from Osaka Seiki)

### [Molding Machine]

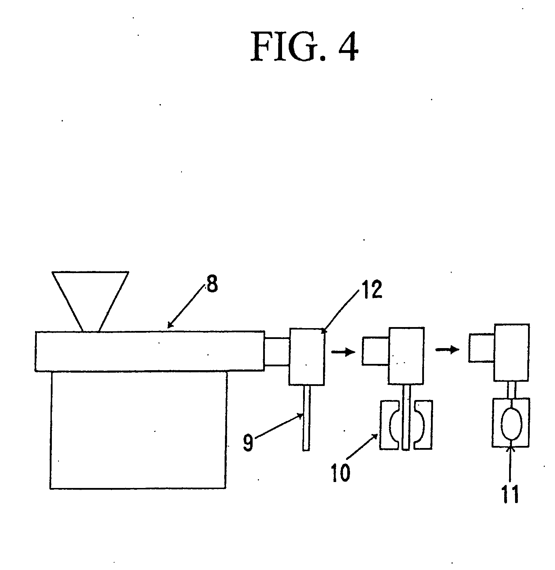

An extrusion blow molding machine: a φ22 mm single-screw extruder as shown in Figure 4 was used (commercially available from Pla-eng). Figure 4 is a schematic view of the extrusion blow molding machine. The φ22 mm single-screw extruder was provided with a die 12, and a parison 9 made of a thermally melted resin composition was extruded from the die 12. Next, the resulting parison 9 was inserted between two split dies having a desired internal shape, and the split dies were clamped together. Thereafter, air was blown into the die 12 from an air mandrel (not shown) of the die 12, thereby giving a blow-molding product having a given shape. In Figure 4, 11 is an air blow provided at the bottom of the split dies 10.

### [Determination of Physical Properties]

### 1. Storage Modulus

The storage modulus was determined by tensile-type dynamic viscoelasticity device commercially available from Rheology under the trade name of DVE-V4 FT Rheospectra (determination temperature: 25°C, shape of cross section of the sample: 1 mm in thickness and 5 mm in width, distance between chucks: 10 mm, strain ratio: 0.03%, frequency: 1 Hz/sine wave, static load: automatic static load control).

### 2. Kink Resistance

A tube was curved and the minimum radius at which kink was not generated was determined by an R gauge.

### 3. Melt Tension

The melt tension was determined by using a capillograph (Shimadzu Corporation), a melt tension determination device in accordance with the following method.

A resin composition was preheated at 230°C for 4 minutes in a cylinder, and thereafter discharged from a capillary (φ1 mm, L/D = 10) with a piston at a rate of 20 mm/min. A strand was taken off at a given speed of 10 m/min, and a load was read off and recorded at a stress gauge via a pulley positioned in the course of take-off to start taking determinations. The melt tension was defined as an average value of the load reading at a point of 20 seconds after the load curve was stabilized.

### Examples 1 to 3

A resin composition was prepared by mixing the polypropylene (Resin 1) and the styrenic elastomer (Resin 4) in a proportion as shown in Table 1, and molded with an extruder (φ40 mm) at a resin temperature of 200°C, to give an endotracheal tube having an inner diameter of φ7 mm and an outer diameter of φ11 mm.

Storage modulus MD in the extrusion direction, storage modulus TD in the circumferential direction, MD/TD and kink resistance of the resulting tube are shown in Table 1.

### Comparative Examples 1 to 4

A resin composition was prepared by mixing the polypropylene (Resin 1) and the styrenic elastomer (Resin 4) in a proportion as shown in Table 1, and molded with an extruder (φ40 mm) at a resin temperature of 200°C, to give an endotracheal tube having an inner diameter of φ7 mm and an outer diameter of φ11 mm.

Storage modulus MD in the extrusion direction of the resulting tube, storage modulus TD in the circumferential direction, MD/TD and kink resistance are shown in Table 1.

**Table 1**

| | Resin 1 (% by weight) | Resin 4 (% by weight) | MD (dyne/cm²) | TD (dyne/cm²) | MD/TD | Kink Resistance (Radius of Curvature: mm) |
|---|---|---|---|---|---|---|
| Ex. 1 | 40 | 60 | 5.15 × 10⁷ (5.15 x 10⁸ dyne/cm²) | 4.41 × 10⁸ (4.41 x 10⁸ dyne/cm²) | 1.17 | 32 |
| Ex. 2 | 30 | 70 | 1.80 × 10⁷ (1.80 x 10⁷dyne/cm²) | 1.63 × 10⁸ (1.63 x 10⁸ dyne/cm²) | 1.10 | 30 |
| Ex. 3 | 20 | 80 | 9.71 × 10⁶ (9.71 x 10⁷dyne/cm²) | 7.93 × 10⁷ (7.93 x 10⁸ dyne/cm²) | 1.22 | 35 |
| Comp. Ex. 1 | 50 | 50 | 8.89 × 10⁷ (8.89 x 10⁸ dyne/cm²) | 6.06 × 10⁸ (6.06 x 10⁸ dyne/cm²) | 1.47 | 50 |
| Comp. Ex. 2 | 60 | 40 | 1.42 × 10⁸ (1.42 x 10⁹dyne/cm²) | 9.23 × 10⁸ (9.23 x 10⁸ dyne/cm²) | 1.54 | 53 |
| Comp. Ex. 3 | 70 | 30 | 2.28 × 10⁸ (2.28 x 10⁹dyne/cm²) | 8.84 × 10⁸ (8.84 x 10⁸ dyne/cm²) | 2.58 | 62 |
| Comp. Ex. 4 | 10 | 90 | 5.87 × 10⁶ (5.87 x 10⁷dyne/cm²) | 4.41 × 10⁷ (4.41 x 10⁷dyne/cm²) | 1.33 | 45 |

It can be seen from the results in Table 1 that since the endotracheal tubes obtained in Examples 1 to 3 have the storage moduli in the extrusion direction of the tube (MD) within the range of 5.0 × 10⁶ - 8.0 × 10⁷ N/m² (5.0 × 10⁷ to 8.0 × 10⁸ dyne/cm²), and MD/TD of not more than 1.3, the endotracheal tubes have small radii of curvature, so that they are excellent in kink resistance.

### Examples 4 to 7

A resin composition was prepared by mixing the polypropylene (Resin 1) and the styrenic elastomer (Resin 4) in a weight ratio of 30/70, and stearic acid monoglyceride or oleic amide was added to the resin composition in a proportion as shown in Table 2. The mixture was molded with an extruder (φ40 mm) at a resin temperature of 200°C, to give an endotracheal tube having an inner diameter of φ7 mm and an outer diameter of φ11 mm. The operability when a suction catheter made of a plasticized-polyvinyl chloride was inserted into the internal of the resulting tube and the printability on the outer surface of the tube were evaluated. The results are shown in Table 2.

**Table 2**

| | Resin Composition (% by wt.) | Stearic Acid Monoglyceride (% by wt.) | Oleic Amide (% by wt.) | Operability | Printability |
|---|---|---|---|---|---|
| Ex. 4 | 99.95 | 0.05 | - | Excellent | ○ |
| Ex. 5 | 99.5 | 0.5 | - | Excellent | ○ |
| Ex. 6 | 99.95 | - | 0.05 | Excellent | ○ |
| Ex. 7 | 99.5 | - | 0.5 | Excellent | ○ |
| Note: 1) Resin Composition: Resin 1/Resin 4 = 30/70 (weight ratio) 2) Operability: The operability was evaluated by inserting a suction catheter into a tube of each example to detect the resistance felt by touch. 3) Printability: An ink for polypropylene was applied to a tube surface, and dried, and thereafter the surface was subjected to scotch tape peeling test. ○: no peeling, ×: peeling | | | | | |

It can be seen from the results shown in Table 2 that the tube produced by adding 0.05 to 0.5% by weight of stearic acid monoglyceride or oleic amide to the resin composition is excellent in the operability of the suction catheter and the printability onto the tube surface.

### Example 8

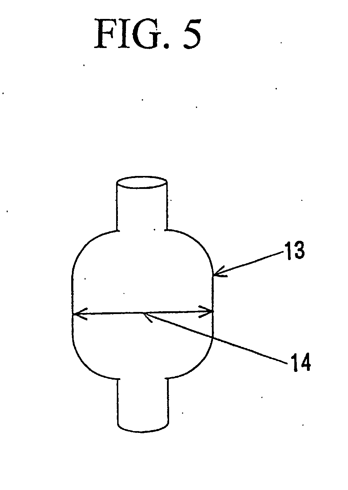

A resin composition was prepared by mixing the polypropylene (Resin 2) and the styrenic elastomer (Resin 4) in a weight ratio of 25/75, and molded by using the extrusion blow molding machine as shown in Figure 4 to give a spindle-like cuff 13 having the shape as shown in Figure 5. The resulting spindle-like cuff 13 had a cuff diameter 14 of about 30 mm and a thickness of 30 to 100 µm.

As to the resulting spindle-like cuff 13, melt tension of the resin composition, blow moldability during molding, and storage modulus are shown in Table 3.

Next, an endotracheal tube was produced by using this cuff and the tube obtained in Example 4. The endotracheal tube was inserted into a trachea of a pig, and the endotracheal sealability was evaluated when the cuff was expanded at a pressure of 25 cm H₂O. The results are also shown in Table 3.

### Example 9

A cuff and an endotracheal tube were produced in the same manner as in Example 8 except that a resin composition was prepared by mixing a polypropylene (Resin 2), a polyethylene (Resin 3) and a styrenic elastomer (Resin 4) in a weight ratio of 12.5/12.5/75. The physical properties of the resulting cuff and the endotracheal tube were evaluated in the same manner as in Example 8. The results are shown in Table 3.

### Comparative Example 5

A cuff and an endotracheal tube were produced in the same manner as in Example 8 except that a resin composition was prepared by mixing a polypropylene (Resin 2) with a styrenic elastomer (Resin 4) in a weight ratio of 50/50. The physical properties of the resulting cuff and the endotracheal tube were evaluated in the same manner as in Example 8. The results are shown in Table 3.

### Comparative Example 6

A cuff and an endotracheal tube were attempted to be produced in the same manner as in Example 8 except that a resin composition was prepared by mixing a polypropylene (Resin 1) with a styrenic elastomer (Resin 4) in a weight ratio of 25/75. However, the cuff was broken during blow-molding, so that a cuff having good physical properties could not be obtained. The results are shown in Table 3.

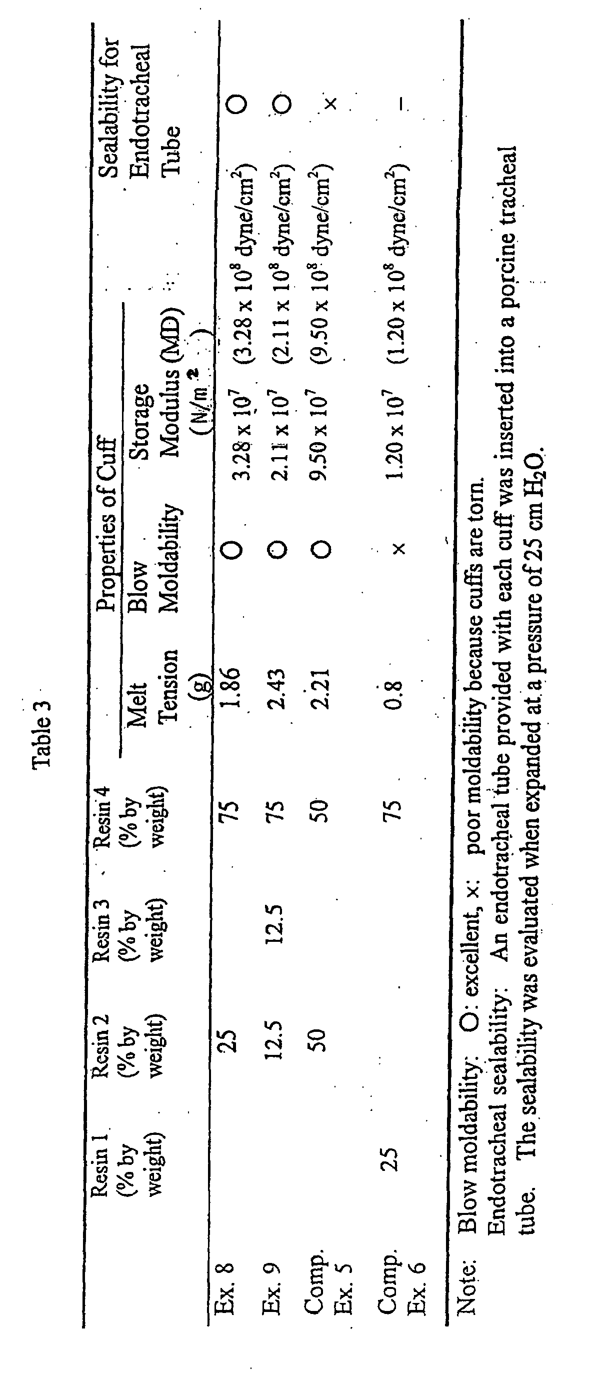

It can be seen from the results shown in Table 3 that the cuffs obtained in Examples 8 and 9 have storage moduli of not more than 5.0 × 10⁷ N/m² (5.0 × 10⁸ dyne/cm²), and melt tension of the resin composition is not less than 1 g, so that the cuffs are excellent in the blow moldability. Also, since the resin compositions are flexible, the resulting cuffs are excellent in its endotracheal sealability when the cuff is expanded at a pressure of 25 cm H₂O in the trachea.

On the other hand, it can be seen in Comparative Example 5 that since a resin composition having a storage modulus greater than 5.0 × 10⁷ N/m² (5.0 × 10⁸ dyne/cm²) is used, the resulting cuff is excellent in blow-moldability, but the cuff has a hard texture, so that the tube is insufficient in the endotracheal sealability during expansion in the trachea.

In addition, it can be seen in Comparative Example 6 that since a resin composition having a melt tension of less than 1 g is used, the cuff is broken during blow-molding, so that a cuff having excellent physical properties cannot be obtained.

### Examples 10 and 11

A cuff and an endotracheal tube were produced in the same manner as in Example 8 except that talc (the Japanese Pharmacopoeia) was added in a ratio as shown in Table 4 to a resin composition prepared by mixing a polypropylene (Resin 2) and a styrenic elastomer (Resin 4) in a weight ratio of 25/75.

Prevention of sticking between the cuff and the main tube of the resulting endotracheal tube, and the surface property of the cuff, i.e. external appearance, were evaluated. The results are shown in Table 4.

**Table 4**

| | Resin Composition (% by wt.) | Talc (% by wt.) | Prevention of Sticking | Surface Property |
|---|---|---|---|---|
| Ex. 10 | 95 | 5 | ○ | ○ |
| Ex. 11 | 80 | 20 | ○ | ○ |
| Note: 1) Resin Composition: Resin 2/Resin 4 = 25/75 (weight ratio) 2) Prevention of Sticking: The prevention of sticking between the cuff and the main tube was evaluated as follows: ○: no sticking, ×: sticking 3) Surface property: The surface property of the cuff was visually examined as follows. ○: excellent surface property, ×: poor surface property | | | | |

It can be seen from the results shown in Table 4 that since the resin compositions constituting the cuffs contain 5 to 20% by weight of talc used in the cuffs of the endotracheal tubes obtained in Example 10 and 11, the cuffs are excellent in prevention of sticking and surface property.

The endotracheal tube of the present invention exhibits excellent kink resistance, slidability and surface property.

## Claims

1. An endotracheal tube comprising a tube obtainable by subjecting a resin composition comprising a styrenic elastomer and a polyolefin to extrusion-molding, wherein the tube has a storage modulus (MD) of 5.0 x 10⁶ to 8.0 x 10⁷ N/m² (5.0 x 10⁷ to 8.0 x 10⁸ dyne/cm²) in the extrusion direction at 25°C, and has a ratio of the storage modulus (MD) in the extrusion direction to a storage modulus (TD) in the circumferential direction (MD/TD) of not more than 1.3 at 25°C, the weight ratio of the polyolefin to the styrenic elastomer being 20/80 to 40/60.

2. An endotracheal tube according to Claim 1, provided with a cuff obtainable by subjecting a resin composition comprising a styrenic elastomer and a polyolefin to blow-molding on an outer peripheral surface, the weight ratio of the styrenic elastomer to the polyolefin being 60/40-80/20, the cuff having a storage modulus of not more than 5.0 x 10⁷ N/m² (5.0 x 10⁸ dyne/cm²) at 25°C, and the resin composition constituting the cuff having a melt tension of not less than 1 g at 230°C.

3. An endotracheal tube according to Claim 2, wherein the resin composition constituting the cuff further comprises an inorganic filler and/or organic cross-linked particles in an amount of 5 to 20% by weight.

4. An endotracheal tube according to Claim 3, wherein the inorganic filler is selected from talc, calcium carbonate and mica, and the organic cross-linked particles are selected from cross-linked acrylic resin beads, cross-linked polyurethane beads and cross-linked polystyrene beads.

5. An endotracheal tube according to any preceding Claim, wherein the styrenic elastomer is a block copolymer of a styrenic polymer block (A) and a hydrogenated conjugated diene polymer block (B).

6. An endotracheal tube according to Claim 5, wherein the hydrogenated conjugated diene polymer block (B) is at least one block selected from a hydrogenated polyisoprene block (B1), a hydrogenated isoprene/butadiene copolymer block (B2) and a hydrogenated polybutadiene block (B3).

7. An endotracheal tube according to Claim 5, wherein the hydrogenated conjugated diene polymer block is a hydrogenated polyisoprene block having a 1,2-bond and 3,4-bond content of 10 to 75% by mol, wherein not less than 70% of carbon-carbon double bonds of the polyisoprene are hydrogenated.

8. An endotracheal tube according to Claim 5, wherein the hydrogenated conjugated diene polymer block is a hydrogenated isoprene/butadiene copolymer block comprising an isoprene/butadiene copolymer obtained by copolymerising isoprene and butadiene in a weight ratio of 5/95 to 95/5, having a 1,2-bond and 3,4-bond content of 20 to 85% by mol, wherein not less than 70% of carbon-carbon double bonds of the isoprene/butadiene copolymer are hydrogenated.

9. An endotracheal tube according to Claim 5, wherein the hydrogenated conjugated diene polymer block is a hydrogenated polybutadiene block having a 1,2-bond and 3,4-bond content of not less than 45% by mol, wherein not less than 70% of carbon-carbon double bonds of the polybutadiene are hydrogenated.

10. An endotracheal tube according to any of Claims 5 to 9, wherein the content of the styrenic polymer block (A) in the block copolymer is 10 to 40% by weight.

11. An endotracheal tube according to any preceding claim wherein the resin composition constituting the tube further comprises a fatty acid amide lubricant and/or a fatty acid monoglyceride lubricant in an amount of 0.05 to 0.5% by weight.

## Patentansprüche

1. Endotrachealröhre, umfassend eine Röhre, die erhältlich ist, indem eine Harzzusammensetzung, umfassend ein Styrolelastomer und ein Polyolefin, Strangpressen unterworfen wird, wobei die Röhre ein Speichermodul (MD) von 5,0 × 10⁶ bis 8,0 × 10⁷ N/m² (5,0 × 10⁷ bis 8,0 × 10⁸ dyn/cm²) in Extrusionsrichtung bei 25°C und ein Speichermodul (MD)-Verhältnis in Extrusionsrichtung zum Speichermodul (TD) in peripherer Richtung (MD/TD) von nicht mehr als 1,3 bei 25°C hat, wobei das Gewichtsverhältnis von Polyolefin zu Styrolelastomer 20:80 bis 40:60 ist.

2. Endotrachealröhre gemäss Anspruch 1, ausgestattet mit einer Manschette, die erhältlich ist, indem eine Harzzusammensetzung, umfassend ein Styrolelastomer und ein Polyolefin, Blasformen auf einer äusseren peripheren Oberfläche unterworfen wird, wobei das Gewichtsverhältnis von Styrolelastomer zu Polyolefin 60:40 bis 80:20 ist, die Manschette bei 25°C ein Speichermodul von nicht mehr als 5,0 × 10⁷ N/m² (5,0 × 10⁸ dyn/cm²) hat und die Harzzusammensetzung, die die Manschette bildet, eine Schmelzspannung von nicht weniger als 1 g bei 230°C hat.

3. Endotrachealröhre gemäss Anspruch 2, wobei die Harzzusammensetzung, die die Manschette bildet, zusätzlich einen anorganischen Füllstoff und/oder organische vernetzte Partikel in einer Menge von 5 bis 20 Gew.% umfasst.

4. Endotrachealröhre gemäss Anspruch 3, wobei der anorganische Füllstoff ausgewählt ist aus Talk, Calciumcarbonat und Glimmer, und die organischen vernetzten Partikel ausgewählt sind aus vernetzten Acrylharzkügelchen, vernetzten Polyurethankügelchen und vernetzten Polystyrolkügelchen.

5. Endotrachealröhre gemäss einem der vorhergehenden Ansprüche, wobei das Styrolelastomer ein Blockcopolymer eines Styrolpolymerblocks (A) und eines hydrogenierten konjugierten Dienpolymerblocks (B) ist.

6. Endotrachealröhre gemäss Anspruch 5, wobei der hydrogenierte konjugierte Dienpolymerblock (B) mindestens ein Block ist, der ausgewählt ist aus einem hydrogenierten Polyisoprenblock (B1), einem hydrogenierten Isopren/Butadien-Copolymerblock (B2) und einem hydrogenierten Polybutadienblock (B3).

7. Endotrachealröhre gemäss Anspruch 5, wobei der hydrogenierte konjugierte Dienpolymerblock ein hydrogenierter Polyisoprenblock mit einer 1,2-Bindung und einem 3,4-Bindungsgehalt von 10 bis 75 mol-% ist, wobei nicht weniger als 70 % der Kohlenstoff-Kohlenstoff-Doppelbindungen des Polyisoprens hydrogeniert sind.

8. Endotrachealröhre gemäss Anspruch 5, wobei der hydrogenierte konjugierte Dienpolymerblock ein hydrogenierter Isopren/Butadien-Copolymerblock ist, umfassend ein Isopren/Butadien-Copolymer, das erhalten wird durch Copolymerisierung von Isopren und Butadien in einem Gewichtsverhältnis von 5:95 bis 95:5 mit einer 1,2-Bindung und einem 3,4-Bindungsgehalt von 20 bis 85 mol-%, wobei nicht weniger als 70 % der Kohlenstoff-Kohlenstoff-Doppelbindungen des Isopren/Butadien-Copolymers hydrogeniert sind.

9. Endotrachealröhre gemäss Anspruch 5, wobei der hydrogenierte konjugierte Dienpolymerblock ein hydrogenierter Polybutadienblock mit einer 1,2-Bindung und einem 3,4-Bindungsgehalt von nicht weniger als 45 mol-% ist, wobei nicht weniger als 70 % der Kohlenstoff-Kohlenstoff-Doppelbindungen des Polybutadiens hydrogeniert sind.

10. Endotrachealröhre gemäss einem der Ansprüche 5 bis 9, wobei der Gehalt des Styrolpolymerblocks (A) in dem Blockpolymer 10 bis 40 Gew.% beträgt.

11. Endotrachealröhre gemäss einem der vorhergehenden Ansprüche, wobei die Harzzusammensetzung, die die Röhre bildet, zusätzlich ein Fettsäureamid-Schmiermittel und/oder ein Fettsäuremonoglycerid-Schmiermittel in einer Menge von 0,05 bis 0,5 Gew.% umfasst.

## Revendications

1. Tube endotrachéal comprenant un tube pouvant être obtenu en soumettant une composition de résine comprenant un élastomère styrénique et une polyoléfine à un moulage par extrusion, le tube possédant un module de conservation (MD) de 5,0 x 10⁶ à 8,0 x 10⁷ N/m² (5,0 x 10⁷ à 8,0 x 10⁸ dyne/cm²) dans la direction d'extrusion à 25°C, et possédant un rapport du module de conservation (MD) dans la direction d'extrusion à un module de conservation (TD) dans la direction circonférentielle (MD/TD) de pas plus que 1,3 à 25°C, le rapport pondéral de la polyoléfine à l'élastomère styrénique étant de 20/80 à 40/60.

2. Tube endotrachéal selon la revendication 1, pourvu d'un ballonnet pouvant être obtenu en soumettant une composition de résine comprenant un élastomère styrénique et une polyoléfine à un moulage par soufflage sur une surface périphérique extérieure, le rapport pondéral de l'élastomère styrénique à la polyoléfine étant de 60/40-80/20, le ballonnet possédant un module de conservation de pas plus que 5,0 x 10⁷ N/m² (5,0 x 10⁸ dyne/cm²) à 25°C, et la composition de résine constituant le ballonnet possédant une tension à l'état fondu de pas moins que 1 g à 230°C.

3. Tube endotrachéal selon la revendication 2, dans lequel la composition de résine constituant le ballonnet comprend en outre une charge inorganique et/ou des particules organiques réticulées dans une quantité de 5 à 20% en poids.

4. Tube endotrachéal selon la revendication 3, dans lequel la charge inorganique est choisie parmi le talc, le carbonate de calcium et le mica, et les particules organiques réticulées sont choisies parmi des perles de résine acrylique réticulée, des perles de polyuréthane réticulé et des perles de polystyrène réticulé.

5. Tube endotrachéal selon l'une quelconque des revendications précédentes, dans lequel l'élastomère styrénique est un copolymère séquencé d'une séquence de polymère styrénique (A) et d'une séquence de polymère diénique hydrogéné conjugué (B).

6. Tube endotrachéal selon la revendication 5, dans lequel la séquence de polymère diénique hydrogéné conjugué (B) est au moins une séquence choisie parmi une séquence de polyisoprène hydrogéné (B1), une séquence de copolymère isoprène/butadiène hydrogéné (B2) et une séquence de polybutadiène hydrogéné (B3).

7. Tube endotrachéal selon la revendication 5, dans lequel la séquence de polymère diénique hydrogéné conjugué est une séquence de polyisoprène hydrogéné ayant une teneur en liaisons 1,2 et en liaisons 3,4 de 10 à 75% en mole, pas moins de 70% des doubles liaisons carbone-carbone du polyisoprène étant hydrogénées.

8. Tube endotrachéal selon la revendication 5, dans lequel la séquence de polymère diénique hydrogéné conjugué est une séquence de copolymère isoprène/butadiène hydrogéné comprenant un copolymère isoprène/butadiène obtenu en copolymérisant de l'isoprène et du butadiène dans un rapport pondéral de 5/95 à 95/5, ayant une teneur en liaisons 1,2 et en liaisons 3,4 de 20 à 85% en mole, pas moins de 70% des doubles liaisons carbone-carbone du copolymère isoprène/butadiène étant hydrogénées.

9. Tube endotrachéal selon la revendication 5, dans lequel la séquence de polymère diénique hydrogéné conjugué est une séquence de polybutadiène hydrogéné ayant une teneur en liaisons 1,2 et en liaisons 3,4 de pas moins que 45% en mole, pas moins de 70% des doubles liaisons carbone-carbone du polybutadiène étant hydrogénées.

10. Tube endotrachéal selon l'une quelconque des revendications 5 à 9, dans lequel la teneur en séquence de polymère styrénique (A) dans le copolymère séquence est de 10 à 40% en poids.

11. Tube endotrachéal selon l'une quelconque des revendications précédentes dans lequel la composition de résine constituant le tube comprend en outre un lubrifiant à base d'amide d'acide gras et/ou un lubrifiant à base de monoglycéride d'acide gras dans une quantité de 0,05 à 0,5% en poids.
